# EUROPEAN PATENT SPECIFICATION

(11) **EP 2 943 795 B1**
(45) Date of publication and mention of the grant of the patent: **24.02.2021**
(21) Application number: 14700090.5
(22) Date of filing: 07.01.2014
(51) Int. Cl.: G01N 33/74

(54) **FASTING LEVELS OF GROWTH HORMONE AS A PREDICTIVE MARKER OF CARDIOVASCULAR RISK**
NÜCHTERN WERTE DES WACHSTUMSHORMONS ALS VORHERSAGEMARKER VOM KARDIOVASKULÄREN RISIKO
TAUX À JEÛN D'HORMONE DE CROISSANCE COMME MARQUEUR DE PRÉDICTION D'UN RISQUE CARDIOVASCULAIRE

(30) Priority: 08.01.2013 EP 13150566
(43) Date of publication of application: 18.11.2015
(73) Proprietor: sphingotec GmbH, 16761 Henningsdorf (DE)
(72) Inventor: BERGMANN, Andreas, 13645 Berlin (DE)
(74) Representative: Kilger, Ute
(86) International application number: PCT/EP2014/050143
(87) International publication number: WO 2014/108396

(56) References cited:
- WO-A2-2007/124439
- US-A1- 2011 263 821
- MAISON P. ET AL.: "Growth hormone as a risk for premature mortality in healthy subjects: Data from the Paris prospective study", BMJ, vol. 31, no. 7138, 11 April 1998 (1998-04-11), pages 1132-1133, XP002699120, cited in the application
- BIDLINGMAIER M. ET AL.: "High-sensitivity chemiluminescence immunoassays for detection of growth hormone doping in sports", CLIN. CHEM., vol. 55, no. 3, 15 January 2009 (2009-01-15), pages 445-453, XP055066670,
- MELANDER O. ET AL.: "Plasma proneurotensin and incidence of diabetes, cardiovascular disease, breast cancer, and mortality", JAMA, vol. 308, no. 14, 10 October 2012 (2012-10-10), pages 1469-1475, XP002699121,
- PALMEIRO C.R. ET AL.: "Growth hormone and the cardiovascular system", CARDIOLOGY IN REVIEW, vol. 20, no. 4, July 2012 (2012-07), pages 197-207, XP009170373,
- BRUNO J.G. ET AL.: "Discrimination of recombinant from natural human growth hormone using DNA aptamers", J. BIOMOL. TECHNIQUES, vol. 22, no. 1, April 2011 (2011-04), pages 27-36, ISSN: 1943-4731

## Description

Subject matter of the present invention is a method for predicting the cardiovascular risk or the overall mortality risk in a subject comprising:
- determining the fasting level of growth hormone (hGH), and/or its isoforms in a bodily fluid obtained from said subject; and
- correlating said fasting level of growth hormone (hGH), and/or its isoforms with a cardiovascular risk or the overall mortality risk, wherein an enhanced level is predictive for an enhanced risk.

Growth Hormone (hGH) is secreted from the anterior pituitary gland and many but not all of its metabolic actions are exerted through IGF-1, {1}. Apart from regulating longitudinal bone growth during childhood and adolescence, hGH exerts metabolic effects throughout the entire life, such as in an anabolic state, acting with insulin to stimulate IGF-1 resulting in protein synthesis and anabolism. In the catabolic state, hGH is stimulating lipolysis and thereby preventing protein degradation and depletion of glycogen stores {1}. In the last decades the role of hGH in the adult cardiovascular system has attracted growing interest, primarily in the situations of hGH deficiency and hGH excess.

It is recognized that hypopituitary adults have an increased mortality in cardiovascular and cerebrovascular diseases and this has been attributed to growth hormone deficiency (GHD) by many {2}, {3}, {4}, but not all {5}. When comparing patients with GHD to healthy individuals, GHD-patients have been shown to have an adverse cardiovascular risk profile with elevated low density lipoprotein cholesterol (LDL-C) {6}, {7}, body mass index (BMI){6} and waist-hip ratio{6}, {8} and decreased high density lipoprotein cholesterol (HDL-C){4}. hGH substitution therapy to these patients has shown a favourable effect on several aspects of the cardiovascular risk profile, e.g. decreases in CRP, body fat, waist-hip-ratio, truncal fat and increased HDL-C {9}, {6}, {10} as well as lower LDL-C and diastolic blood pressure {11}. Even though effects on glucose homeostasis may be negative {11}, {1}, it is with this background tentative to assume that hGH-substitution is beneficial, however to date, there are no large placebo-controlled, randomized prospective studies to demonstrate the effect of hGH-substitution on cardiovascular morbidity or mortality.

At the other end of the spectrum, patients with acromegaly, who are suffering from excess secretion of growth hormone, also exhibit elevated mortality rates in cardiovascular and cerebrovascular diseases {12}. Contributing factors to this mortality are found in the higher prevalence of hypertension, diabetes, arrhythmias and cardiomyopathy with hypertrophy of the heart {13}, {14}. Inhibiting the hGH secretion with somatostatin analogs in these patients has shown beneficial effects on heart structure and performance {15}. Further demonstration of the detrimental effects of hGH excess is made by the study by Takala *et al.* in 1999 where administration of high-dose hGH to critically ill patients in a intensive care unit doubled the mortality rates compared to placebo {16}.

Most of the literature concern patients with either excess or deficiency of hGH. However the prospective study of French policemen started in the late 60's and early 70's with an 18 year follow-up, examined fasting values of hGH and the risk of premature mortality. Their findings show that higher levels of hGH after a 2h oral glucose tolerance test are associated with an elevated risk of premature mortality of all causes and of cardiovascular disorders, fasting hGH (measured with standard hGH test was not significant for prediction of mortality {17}. Using a 0.5ng/ml cut off value, Maison *et al.* found an increase of 18 year cardiac mortality with a Hazard Risk of 1.79.

Accurate interpretation of plasma hGH concentration is hampered by the fact that hGH is released in pulses over the majority of the day and in the morning hours, when plasma concentration is relatively stable {32}, {33}, {34}, hGH concentrations are low and sometimes undetectable using conventional hGH assays.

Described in the present disclosure is an ultra sensitivity assay for plasma hGH measurements (hGH), with accurate measurements also at low hGH plasma concentrations (e.g. assay sensitivity 2 pg/ml). Subject matter of the present invention is the correlation of the development of cardiovascular morbidity and mortality with hGH in fasted plasma in a subject. Subject matter of the present invention is the relation of hGH in fasted plasma in a subject and the risk of coronary artery disease (CAD), stroke, all-cause (total) mortality, and cardiovascular (CVD) mortality.

Subject matter of the present invention is a method for predicting the cardiovascular risk or the total mortality risk in a subject comprising:
▪ determining the fasting level of growth hormone (hGH), and/or its isoforms in a bodily fluid obtained from said subject; and
▪ correlating said fasting level of growth hormone (hGH), and/or its isoforms with a cardiovascular risk or the total mortality risk, wherein an enhanced level is predictive for an enhanced risk.

Cardiovascular risk or total mortality risk means the risk of getting an event due to cardiovascular reasons or the risk of dying because of all causes within 2,5 years.

Subject matter of the present invention is method for predicting the cardiovascular risk or the total mortality risk within 2,5 years, in a subject comprising:
▪ determining the fasting level of growth hormone (hGH), and/or its isoforms in a bodily fluid obtained from said subject; and
▪ correlating said fasting level of growth hormone (hGH), and/or its isoforms with a cardiovascular risk or the total mortality risk, wherein an enhanced level is predictive for an enhanced risk,
wherein the cardiovascular risk is defined as an adverse event or the total mortality risk and wherein the risk of said adverse event or the total mortality risk is predicted within 2,5 years after taking said bodily fluid from said subject.

In one embodiment said subject is a healthy subject at the time of measurement or a subject that does not have a cardiovascular disease at the time of measurement. In one embodiment said subject has never had a stroke and/or myocardial infarction and /or acute heart failure at the time of measurement. In one embodiment said subject has never had Coronary artery disease (CAD) and / or ischemic heart disease and/or percutaneous coronary intervention (PCI) or coronary artery by-pass grafting (CABG) at the time of measurement.

Total mortality risk is the risk of dying because of all causes, is the risk of death within a certain period of time.

In a specific embodiment of the invention the cardiovascular risk is defined as an event selected from the group comprising stroke, CVD (cardiovascular disease) mortality and Coronary artery disease (CAD) wherein the latter comprises fatal or nonfatal myocardial infarction, death due to ischemic heart disease, percutaneous coronary intervention (PCI) or coronary artery by-pass grafting (CABG). In one embodiment CVD (cardiovascular disease) mortality is the mortality due to myocardial infarction, acute heart failure or stroke. This means that CVD (cardiovascular disease) mortality is death due to a cardiovascular disease. In a specific embodiment CVD (cardiovascular disease) is selected from the group comprising myocardial infarction, acute heart failure and stroke.

The cardiovascular risk or the total mortality risk is a short term prediction of said risk.

That means the risk of a subject of getting an event associated to a cardiovascular risk or of dying because of all causes (total mortality) within a short term is predicted wherein said short term means within 2,5 years.

It is the surprising finding of the present invention that a short-term prognosis of the cardiovascular risk or the total mortality risk is possible by determining the fasting level of growth hormone (hGH), and/or its isoforms in a bodily fluid. Short-term means within 2,5 years after taking said bodily fluid from said subject.

The prediction of CVD mortality is a short term prediction. That means the risk of a subject of dying because of myocardial infarction, acute heart failure or stroke within a short term is predicted wherein said short term means within 2,5 years.

In a more specific embodiment the prediction of CVD mortality is a short term prediction for a male subject. That means in a more specific embodiment the risk of a male subject of dying because of myocardial infarction, acute heart failure or stroke within a short term is predicted wherein said short term means within 2,5 years.

Isoforms of growth hormone (hGH) may be selected from the group comprising hGH isoform 1 (22KD), hGH isoform 2, hGH isoform 3, and hGH isoform 4.

### Sequences of the isoforms:

SEQ ID NO.1: hGH Isoform 1 (22KD)











SEQ ID NO.2: hGH Isoform 2

MATGSRTSLLLAFGLLCLPWLQEGSAFPTIPLSRLFDNAMLRAHRLHQLAFDTYQEFN
PQ

TSLCFSESIPTPSNREETQQKSNLELLRISLLLIQSWLEPVQFLRSVFANSLVYGASDSN

VYDLLKDLEEGIQTLMGRLEDGSPRTGQIFKQTYSKFDTNSHNDDALLKNYGLLYCF
RKD

MDKVETFLRIVQCRSVEGSCGF

SEQ ID NO.3: hGH Isoform 3

MATGSRTSLLLAFGLLCLPWLQEGSAFPTIPLSRLFDNAMLRAHRLHQLAFDTYQEFE
EA

YIPKEQKYSFLQNPQTSLCFSESIPTPSNREETQQKSNLELLRISLLLIQTLMGRLEDGS

PRTGQIFKQTYSKFDTNSHNDDALLKNYGLLYCFRKDMDKVETFLRIVQCRSVEGSC
GF

SEQ ID NO.4: hGH Isoform 4

MATGSRTSLLLAFGLLCLPWLQEGSAFPTIPLSRLFDNAMLRAHRLHQLAFDTYQEFE
EA

YIPKEQKYSFLQNPQTSLCFSESIPTPSNREETQQKSNLELLRISLLLIQSWLEPVQIFK

QTYSKFDTNSHNDDALLKNYGLLYCFRKDMDKVETFLRIVQCRSVEGSCGF

Fasting levels of growth hormone (hGH) means the level of growth hormone (hGH) determined in blood, serum or plasma of fastened subjects. (12 h no food uptake prior sample taking). Fastened subject means a subject that had 12 h prior sample taking no food up-take.

The term "subject" as used herein refers to a living human or non-human organism. Preferably herein the subject is a human subject. The subject may be healthy or diseased if not stated otherwise. The term "elevated level" means a level above a certain threshold level.

A bodily fluid may be selected from the group comprising blood, plasma, serum, saliva, urine, and cerebrospinal fluid. In a specific embodiment said bodily fluid is selected from the group comprising blood, plasma, and serum.

Assay sensitivity may be defined by the "analytical" assay sensitivity that is defined as hGH concentration (standard curve readout) at a median signal of e.g. 20 determinations of hGH depleted sample + 2 standard deviations (SD).

Alternatively, the assay sensitivity may be defined by the "functional" assay sensitivity, which reflects more the limit of detection in a clinical routine. The functional assay sensitivity is defined as determination of the inter assay coefficient of variation (CV) of plasma (patient samples) in a variety of individual samples with different hGH concentrations. The functional assay sensitivity is the lowest hGH concentration with an inter assay CV of less than 20%.

Assay sensitivities may depend upon the used method of calibration and the used hGH assay. Different hGH assays may detect different sets of hGH isoforms, leading to different quantitative results meaning leading to different thresholds and sensitivity needs depending on the assay and the calibration method used,

To overcome this possible heterogeneity of different hGH assays, assay calibrations may be corrected by correlating plasma hGH values of individual subjects. If the slope of the correlation curve is different from 1, the hGH values (the calibration) can be corrected by the differential factor leading to a comparable calibration, comparable sensitivity analysis and comparable thresholds.

The above correction may be performed in order to compare the results obtained by different assays.

The assay described in example 1 predominantly recognizes hGH isoform 1 and is calibrated by the recombinant Human Growth Hormone (NIBSC code 98/574, National Institute for Biological Standards and Control, Herfordshire, UK).

In a specific method according to the invention said fasting level of growth hormone (hGH), and/or its isoforms in a bodily fluid is determined by an ultrasensitive assay having an analytical assay sensitivity (that is defined as hGH concentration at a median signal of 20 determinations of hGH depleted sample + 2 standard deviations (SD)) of less than 100 pg/ml, preferred less than 50 pg/ml, preferred less than 30 pg/ml, preferred less than 20 pg/ml, preferred less than 10 pg/ml, preferred less than 5 pg/ml, preferred 2 pg/ml. Measurement of hGH is expressed in ng/ml (1ng/ml=2.6 mU/L).

Alternatively, the ultrasensitive assay having a functional assay sensitivity (see above) of less than 400 pg/ml, preferred less than 200 pg/ml, preferred less than 120 pg/ml, preferred less than 80 pg/ml, preferred less than 40 pg/ml, preferred less than 20 pg/ml, preferred 10 pg/ml most preferred less than 8,5 pg/ml. Measurement of hGH is expressed in ng/ml.

For immunization and for calibration we used recombinant Human Growth Hormone (NIBSC code 98/574, National Institute for Biological Standards and Control, Herfordshire, UK)

In one embodiment of the invention said assay is conducted as described in Example 1.

Said assay may be selective for one specific hGH isoform or maybe specific for and determines more than one or all secreted isomers of hGH selected from the group comprising hGH Isoform 1, Isoform 2, Isoform 3, and Isoform 4 (see SEQ ID NO. 1-4) in said bodily fluid.

In one embodiment of the method according to the invention said a method is performed more than once in order to monitor the cardiovascular risk in a subject or in order to monitor the course of treatment.

In one embodiment of the method according to the invention said monitoring is performed in order to evaluate the response of said subject to preventive and/or therapeutic measures taken.

In one embodiment of the method according to the invention said method is used in order to stratify said subjects into risk groups and/or to reclassify said subjects on top of classical risk markers.

In one embodiment of the invention said subject is male and the threshold is from above 400 pg/ml, preferably 340 pg/ml, to 100 pg/ml, preferably 60 pg/ml, wherein a fasting level of growth hormone (hGH), and/or its isoforms above a threshold above 400 pg/ml, preferably 340 pg/ml is predictive for a high risk and a fasting level below a threshold of 100 pg/ml, preferably 60 pg/ml is predictive for a low risk.

In another embodiment of the invention said subject is female and the threshold is from 3150 pg/ml, preferably to 400 pg/ml, wherein a fasting level of growth hormone (hGH), and/or its isoforms above a threshold of 3150 pg/ml is predictive for a high risk and a fasting level below a threshold of preferably 400 pg/ml is predictive for a low risk.

In one embodiment of the invention said cardiovascular risk is selected myocardial infarction, acute heart failure and stroke.

In another embodiment at least one clinical parameter is additionally determined wherein said clinical parameter is selected from the group comprising: age, presence of diabetes mellitus, current smoking.

In a specific embodiment of the methods of the present invention additionally at least one further biomarker is determined in the bodily fluid of said subject and correlated with said cardiovascular risk, wherein said additional biomarker is selected from the group comprising: pro-Neurotensin 1-117 (PNT 1-117), C-reactive protein (CRP), pro-brain natriuretic peptide 1-108 (pro-BNP) 1-108, Pro-BNP, BNP, Pro Atrial Natriuretic Peptide 1-98 (proANP-N-terminal fragment), Pro-ANP, Adrenomedullin, pro-adrenomedullin (proADM) 24-71, ProADM 127-164, pro- Atrial Natriuretic Peptide (pro-ANP) and fragments thereof having at least a length of five amino acids, ST-2, GDF15, Galectin-3 Copeptin..

Subject matter according to the present invention is a method wherein the fasting level of growth hormone (hGH), and/or its isoforms is determined by using a binder to growth hormone (hGH), and/or its isoforms. The additional above mentioned further biomarkers may be determined by using a binder to said biomarkers or their fragments as outlined above.

In one embodiment of the invention said binder is selected from the group comprising an antibody, an antibody fragment or a non-Ig-Scaffold binding to growth hormone (hGH), and/or its isoforms.

According to the invention the diagnostic binder is selected from the group consisting of antibodies e.g. IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains, *e.g*. Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines.

In a specific embodiment the level of the above biomarker or fragments thereof are measured with an assay using binders selected from the group comprising aptamers, non-Ig scaffolds as described in greater detail below binding to hGH or fragments thereof.

Binder that may be used for determining the level of any of the above biomarkers or fragments thereof exhibit an affinity constant to the binding region of any of the above biomarkers of at least 10⁷ M⁻¹, preferred 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention. Binding affinity may be determined using the Biacore method, offered as service analysis e.g. at Biaffin, Kassel, Germany (http://www.biaffin.com/de/).

### Affinity Constants.

To determine the affinity of the antibodies, the kinetics of binding of hGH to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare) {59}.

In addition to antibodies other biopolymer scaffolds are well known in the art to complex a target molecule and have been used for the generation of highly target specific biopolymers. Examples are aptamers, spiegelmers, anticalins and conotoxins. Non-Ig scaffolds may be protein scaffolds and may be used as antibody mimics as they are capable to bind to ligands or antigenes. Non-Ig scaffolds may be selected from the group comprising tetranectin-based non-Ig scaffolds (*e.g.* described in US 2010/0028995), fibronectin scaffolds (*e.g.* described in EP 1266 025; lipocalin-based scaffolds (*e.g*. described in WO 2011/154420); ubiquitin scaffolds (*e.g.* described in WO 2011/073214), transferring scaffolds (*e.g.* described in US 2004/0023334), protein A scaffolds (*e.g*. described in EP 2231860), ankyrin repeat based scaffolds (*e.g*. described in WO 2010/060748), microproteins preferably microproteins forming a cystine knot) scaffolds (*e.g*. described in EP 2314308), Fyn SH3 domain based scaffolds (*e.g.* described in WO 2011/023685) EGFR-A-domain based scaffolds (*e.g.* described in WO 2005/040229) and Kunitz domain based scaffolds (*e.g*. described in EP 1941867).

Subject of the present invention is also a method for predicting the cardiovascular risk, the mortality because of cardiovascular events or the total mortality risk in a subject according to any of the preceding embodiments, wherein the level of growth hormone (hGH), and/or its isoforms in a bodily fluid obtained from said subject either alone or in conjunction with other prognostically useful laboratory or clinical parameters is used which may be selected from the following alternatives:
- Comparison with the median of the level of hGH or fragments thereof of at least 5 amino acids in a bodily fluid obtained from said subject in an ensemble of predetermined samples in a population of "healthy" or "apparently healthy" subjects,
- Comparison with a quantile of the level of hGH or fragments thereof of at least 5 amino acids in a bodily fluid obtained from said subject in an ensemble of predetermined samples in a population of "healthy" or "apparently healthy" subjects,
- Calculation based on Cox Proportional Hazards analysis or by using Risk index calculations such as the NRI (Net Reclassification Index) or the IDI (Integrated Discrimination Index).

In a specific embodiment of the methods according to the present invention said subject is female and one further biomarker is determined in addition to hGH and its isomers in the bodily fluid of said female subject and correlated with said cardiovascular risk, wherein said additional biomarker is pro-Neurotensin (PNT) and fragments thereof having at least a length of five amino acids.

In one embodiment of the invention it may be a so-called POC-test (point-of-care), that is a test technology which allows performing the test within less than 1 hour near the patient without the requirement of a fully automated assay system. One example for this technology is the immunochromatographic test technology.

In one embodiment of the invention such an assay is a sandwich immunoassay using any kind of detection technology including but not restricted to enzyme label, chemiluminescence label, electrochemiluminescence label, preferably a fully automated assay. In one embodiment of the invention such an assay is an enzyme labeled sandwich assay. Examples of automated or fully automated assay comprise assays that may be used for one of the following systems: Roche Elecsys®, Abbott Architect®, Siemens Centauer®, Brahms Kryptor®, Biomerieux Vidas®, Alere Triage®.

A variety of immunoassays are known and may be used for the assays and methods of the present invention, these include: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immuno-chromotography assays.

In one embodiment of the invention at least one of said two binders is labeled in order to be detected.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

In a preferred embodiment said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person {23}.

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in {24}. Preferred chemiluminescent dyes are acridiniumesters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably more than 10⁸ M⁻¹.

In the context of the present invention, "binder molecules" are molecules which may be used to bind target molecules or molecules of interest, *i.e.* analytes (*i.e.* in the context of the present invention PCT and fragments thereof), from a sample. Binder molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, binder molecules may for instance be selected from the group comprising a nucleic acid molecule, a carbohydrate molecule, a PNA molecule, a protein, an antibody, a peptide or a glycoprotein. Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

Chemiluminescent label may be acridinium ester label, steroid labels involving isoluminol labels and the like.

Enzyme labels may be lactate dehydrogenase (LDH), creatinekinase (CPK), alkaline phosphatase, aspartate aminotransferace (AST), alanine aminotransferace (ALT), acid phosphatase, glucose-6-phosphate dehydrogenase and so on.

In one embodiment of the invention at least one of said two binders is bound to a solid phase as magnetic particles, and polystyrene surfaces.

In one embodiment of the assays for determining the level of growth hormone (hGH), and/or its isoforms in a sample according to the present invention such assay is a sandwich assay, preferably a fully automated assay. It may be an ELISA fully automated or manual. It may be a so-called POC-test (point-of-care). Examples of automated or fully automated assay comprise assays that may be used for one of the following systems: Roche Elecsys®, Abbott Architect®, Siemens Centauer®, Brahms Kryptor®, Biomerieux Vidas®, Alere Triage®. Examples of test formats are provided above.

In one embodiment of the assays for determining the level of growth hormone (hGH), and/or its isoforms in a sample according to the present invention at least one of said two binders is labeled in order to be detected. Examples of labels are provided above.

In one embodiment of the assays for determining the level of growth hormone (hGH), and/or its isoforms in a sample according to the present invention at least one of said two binders is bound to a solid phase. Examples of solid phases are provided above.

In one embodiment of the assays for determining the level of growth hormone (hGH), and/or its isoforms in a sample according to the present invention said label is selected from the group comprising chemiluminescent label, enzyme label, fluorescence label, radioiodine label.

Further disclosed herein is a kit comprising an assay as described in the present disclosure, wherein the components of said assay may be comprised in one or more container.

According to the present invention said subject may have a co-morbidity, that may be a disease or an event selected from the group comprising atherosclerosis, high blood pressure, heart failure, myocardial infarction.

In a preferred embodiment of the invention the fasting level said of growth hormone (hGH), and/or its isoforms is measured with an immunoassay. In an even more preferred embodiment said immunoassay comprises at least one antibody, preferably two antibodies, having a binding affinity of 10⁻⁸M or less.

Further described in the present disclosure is also an ultra sensitive (us-hGH) assay comprising at least one monospecific binder, preferably two monospecific binders, having a binding affinity of 10⁻⁸M or less to hGH and/ or its isoforms and wherein ultra sensitive assay has an analytical assay sensitivity of less than 100 pg/ml, preferred less than 50 pg/ml, preferred less than 30 pg/ml, preferred less than 20 pg/ml, preferred less than 10 pg/ml, preferred less than 5 pg/ml, preferred 2 pg/ml.

In one specific aspect of the ultrasensitive hGH assay said monospecific binder is a monoclonal antibody.

The ultrasensitive hGH assay as described herein is used in the method according to the present invention.

### EXAMPLES

### Example 1, us-hGH Assay

### Chemicals

If not stated otherwise, chemicals were obtained at p.a. grade from Merck (Darmstadt, Germany).

### Antigen

For immunization and for calibration we used recombinant Human Growth Hormone (NIBSC code 98/574, National Institute for Biological Standards and Control, Herfordshire, UK)

### Development of Antibodies

Mouse monoclonal antibodies against hGH were developed by UNICUS (Karlsburg, Germany).

### The antibodies were generated according to the following method:

A BALB/c mouse were immunized with 100 µg hGH at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intra venous injection.

Splenocytes from the immunized mouse and cells of the myeloma cell line SP2/0 were fused with 1 ml 50 % polyethylene glycol for 30 s at 37°C. After washing, the cells were seeded in 96-well cell culture plates. Hybrid clones were selected by growing in HAT medium [RPMI 1640 culture medium supplemented with 20 % fetal calf serum and HAT-supplement]. After two weeks the HAT medium is replaced with HT Medium for three passages followed by returning to the normal cell culture medium.
The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined. {60}, {61}.

### Monoclonal antibody production

We selected 5 antibodies for further investigations.

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Labelling and coating of antibodies.

All antibodies were labelled with acridinium ester according the following procedure:
Labelled compound (tracer): 100 µg (100 µl) antibody (1 mg/ml in PBS, pH 7.4), was mixed with 10 µl?? Acridinium NHS-ester (1 mg/ml in acetonitrile, InVent GmbH, Germany) (EP 0353971) and incubated for 20 min at room temperature. Labelled antibody was purified by gel-filtration HPLC on Bio-Sil SEC 400-5 (Bio-Rad Laboratories, Inc., USA) The purified labelled antibody was diluted in (300 ?mmol/l potassiumphosphate, 100 mmol/l NaCl, 10 ?mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluminescence was measured by using an AutoLumat LB 953 (Berthold Technologies GmbH & Co. KG).

Solid phase antibody (coated antibody):
Solid phase: Polystyrene tubes (Greiner Bio-One International AG, Austria) were coated (18 h at room temperature) with antibody (1.5 µg antibody/0.3 ml 100 mmol/l NaCl, 50 mmol/l Tris/HCl, pH 7.8). After blocking with 5 % bovine serum albumin, the tubes were washed with PBS, pH 7.4 and vacuum dried.

### hGH Immunoassay:

50 µl of sample (or calibrator) was pipetted into coated tubes, after adding labeled antibody (200ul), the tubes were incubated for 2 h at 18-25 °C. Unbound tracer was removed by washing 5 times (each 1 ml) with washing solution (20 mmol/l PBS, pH 7.4, 0.1 % Triton X-100). Tube-bound labelled antibody was measured by using the LB 953 . Using a fixed concentration 1ng/ml of hGH. The signal (RLU at 1ng hGH /ml) to noise (RLU without us-hGH) ratio of different antibody combinations is given in table 1. All antibodies were able to generate a sandwich complex with any other antibody. The antibody pair with strongest signal to noise ratio (best sensitivity) was subsequently used to perform the us-hGH-immunoassay: hGH G12 antibody was used as coated tube antibody and hGH H4 antibody was used as labelled antibody.

**Table 1: Results of noise to ratio determinations between different pairs of hGH antibodies.**

| hGH antibody | Solid phase antibody | H2 | H8 | G12 | H4 | D7 |
|---|---|---|---|---|---|---|
| Labelled antibody | | | | | | |
| H2 | | / | 9.665 | 11.005 | 9.259 | 10.102 |
| H8 | | 8.512 | / | 7.833 | 8.446 | 6.384 |
| G12 | | 10.112 | 9.846 | / | 10.905 | 7.751 |
| H4 | | 11.213 | 8.675 | 12.225 | / | 6.843 |
| D7 | | 2.488 | 2.761 | 3.954 | 2.713 | / |

### Calibration:

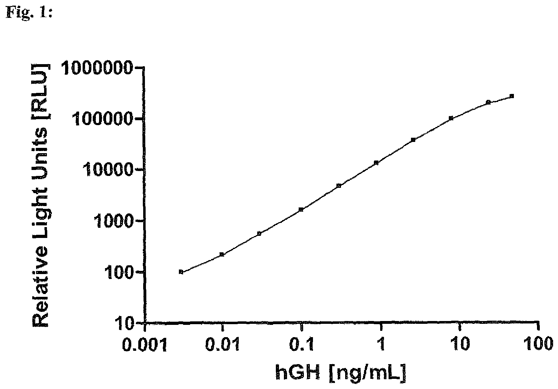

The assay was calibrated, using dilutions of recombinant hGH (WHO International Standard, NIBSC code 98/574), diluted in 20 mM K2PO4, 6 mM EDTA, 0,5% BSA, 50uM Amastatin, 100uM Leupeptin, pH 8.0. (Fig. 1)

### Assay specifications

The analytical assay sensitivity (mean relative light units of 20 determinations of hGH free sample plus 2 S.D.) was 2pg/ml of hGH and the functional assay sensitivity (see above) was 8,5 pg/ml. Recovery and dilution was > 85% intra measurement range of 5-10.000 pg/ml hGH. The coefficient of correlation of N=997 samples between the us-hGH assay and a hGH assay specific for recombinant hGH (22KD) was r=0.98 and a r of 0.95 was found for an assay recognizing preferentially hGH isoforms naturally produced by the pituitary {20}. These dataindicating the suitability of all hGH isoform measurements within the present invention.

### Example 2 Population Study

### Subjects and methods

### Study population

The Malmö diet and cancer study (MDC) is a population-based, prospective epidemiologic cohort of 28 449 individuals examined between 1991 and 1996 {18}. From this cohort a random sample, examined between November 1991 and February 1994 (n=6103) were included in the MDC cardiovascular cohort (MDC-CC), with the primary aim to study the epidemiology of carotid artery disease {19}. When excluding individuals lacking values from fasting plasma samples and thus missing data on prevalence of diabetes mellitus or fasting values of HDL-C, LDL-C or hGH, 4453 persons remained and comprise the primary study cohort.

All participants provided written consent and the study was approved by the ethical committee at Lund University, Lund, Sweden.

### Clinical examination and assays

Participants underwent a medical history, physical examination and laboratory assessment. Blood pressure was measured with a mercury-column sphygmomanometer after 10 minutes rest in the supine position. Waist circumference was measured at the level of the umbilicus. Current cigarette smoking was defined as any use within the last year, and was surveyed through a self-administered questionnaire. The same questionnaire in combination with a diary was used to record prevalence of anti-hypertensive medication at baseline. Diabetes Mellitus was defined as either self-report of a physician diagnosis, use of diabetes medication or fasting whole blood glucose higher than 6.0mmol/L (109 mg/dL).

All samples of plasma and whole-blood were obtained after overnight fasting and samples were drawn between 7.30 am and 9.00 am after 15 minutes of rest in the supine position. Levels of HDL-C and total cholesterol were measured according to standard procedures at the Department of Clinical Chemistry, University Hospital of Malmö. The levels of LDL-C were calculated according to the Friedewald formula.

hGH levels were measured in stored fasting plasma samples, which had been frozen to -80°C immediately at the MDC-CC baseline examination. The measurement was made with an ultra-sensitivity chemiluminescence immunoassay (see example 1 us-hGH assay). The detection limit was 2 pg/ml, functional assay sensitivity was 8,5 pg/ml. Measurement of hGH is expressed in ng/ml (1ng/ml=2.6 mU/L).

**Table 2. Clinical characteristics of the study population.**

| | Male | Female |
|---|---|---|
| Number (% of whole cohort) | 1873 (42.1) | 2580 (57.9) |
| Age, mean (SD), y | 57.9 (6.0) | 57.6 (5.9) |
| Systolic blood pressure, mean (SD), mmHg | 144(19) | 141 (19) |
| Body Mass Index, Mean (SD), kg/m2 | 26.2 (3.5) | 25.5 (4.2) |
| Antihypertensive therapy, No. (%) | 345 (18.4) | 402(15.6) |
| Diabetes Mellitus, No. (%) | 216 (11.5) | 166 (6.4) |
| LDL-C, mean (SD), mmol/L | 4.11 (0.90) | 4.20 (1.04) |
| HDL-C, mean (SD), mmol/L | 1.22 (0.30) | 1.51 (0.37) |
| Current smokers, No. (%) | 512 (27.3) | 661 (25.6) |
| Growth Hormone, median (IQR), ng/mL | 0.11 (0.06-0.34) | 1.22 (0.39-3.15)* |

| | | |
|---|---|---|
| *Significant difference vs males (p<.001). P for difference in other variables not calcuated. Abbreviations: BMI, Body Mass Index; LDL-C, Low-density lipoprotein cholesterol; HDL, High-density lipoprotein cholesterol | | |

### Distribution of hGH in males and females

**Table 3: frequence distribution of hGH in males and females Range of hGH-values in quartiles for all-cause and CVD mortality.**

| **Males** | Q1 | Q2 | Q3 | Q4 |
|---|---|---|---|---|
| Minimum hGH | 0.02 | 0.06 | 0.12 | 0.34 |
| Maximum hGH | 0.05 | 0.11 | 0.33 | 23.94 |
| Minimum Ln hGH | -3.91 | -2.81 | -2.12 | -1.10 |
| Maximum Ln hGH | -3.00 | -2.21 | -1.11 | 3.18 |

| **Females** | | | | |
|---|---|---|---|---|
| Minimum hGH | 0.01 | 0.40 | 1.22 | 3.15 |
| Maximum hGH | 0.39 | 1.21 | 3.14 | 40.60 |
| Minimum Ln hGH | -4.61 | -0.92 | 0.20 | 1.15 |
| Maximum Ln hGH | -0.94 | 0.19 | 1.14 | 3.70 |

The lowest observed hGH concentrations were 0.02 ng/ml in males and 0,01 ng/ml in females, indicating the suitability of the used us-hGH test (analytical sensitivity: 0.002 ng/ml (2 pg/ml), functional assay sensitivity 0.0085ng/ml (8,5pg/ml)) for detecting fasting hGH in all subjects. Cut off values for CVD-mortality in males may be above 0,34 ng/ml for high risk subjects and below 0,06 ng/ml for low risk.??

### Clinical end points

We examined 4 primary outcomes: Coronary artery disease (CAD), stroke, cardiovascular mortality and total mortality. The endpoints were retrieved through record linkage of the personal identification number of each Swedish individual and the Swedish Hospital Discharge Register (SHDR), the Swedish Cause of Death Register (SCDR), the Stroke in Malmö Register and the Swedish Web-system for Enhancement and Development of Evidence-based care in Heart disease Evaluated According to Recommended Therapies (SWEDEHEART). The use of these registries for classification of outcomes has been previously validated and the sensitivity for detecting events such as myocardial infarction has been shown to exceed 90% {21}, {22}, {23}. CAD was defined as fatal or nonfatal myocardial infarction, death due to ischemic heart disease, percutaneous coronary intervention (PCI) or coronary artery by-pass grafting (CABG), whichever came first, on the basis of *International Classification of Diseases,* 9^{th} and 10^{th} revisions (ICD-9 and ICD-10) codes 410 and 121 respectively in the SHDR or SCDR, codes 412 and 414 (ICD-9) or 122,123 and 125 (ICD-10) of the SCDR. Stroke was defined as fatal or nonfatal stroke on the basis of codes 430, 431, 434 and 436 (ICD-9) or 160, 161, 163 and 164 (ICD-10). Cardiovascular mortality was defined on the basis of (ICD-9) codes 390-459 or (ICD-10) codes 100-199 in the SCDR. Follow up for outcomes extended to June 30, 2009.

### Statistical analyses

Fasting values of hGH exhibited a right-skewed distribution and were transformed into the natural logarithm before any analysis. To determine if hGH displayed any correlation with traditional cardiovascular risk factors {24}, cross sectional analyses were performed at baseline using linear regression models with hGH as the dependent variable and age, gender, current smoking, anti-hypertensive medication, diabetes mellitus and the standardized values for: systolic blood pressure, BMI, HDL-C and LDL-C as independent variables entered either separately (crude) or simultaneously (multivariate adjusted).

Multivariable Cox proportional hazard models were performed to examine the association between hGH and incidence of cardiovascular events and mortality. In the analyses of CAD and stroke, we excluded individuals who had a history of CAD (n=94) or stroke (n=35) before baseline in respective analysis. All models were adjusted for age, sex, systolic blood pressure, use of antihypertensive medication, current smoking, diabetes mellitus, BMI and levels of LDL-C and HDL-C. We confirmed that the proportionality of hazards assumption was met using Schoenfeld residuals (i.e. a test of non-zero slope in a generalized linear regression of the scaled Schoenfeld residuals on time). Hazard ratios (HR) for hGH were expressed per 1-SD increment of natural logarithm of hGH. In order to establish if there were any significant gender differences a sex interaction test was performed.

To obtain Kaplan-Meier curves, the cohort was split into gender-specific quartiles.

Table 3 shows the cut off concentrations of the different quartiles in males and females. Cut off concentrations may be adapted if hGH assays are used which recognize different sets of one or more hGH isoforms.

To evaluate possible different results by using different assays, recognizing different sets of hGH isoforms, we compared the assay (antibodies generated against recombinant hGH (isoform1) described in example 1 with an assay preferentially recognizing isoforms 2-4 (Pit-hGH assay, {29}. hGH -results from 997 healthy subjects were correlated, the coefficient of correlation was 0,95 (r=0,95), indicating almost identical results using assays binding to different hGH isoforms.

To evaluate if hGH could be used to reclassify risk we followed methods previously described to calculate a net reclassification improvement (NRI) {25}. We used multivariable risk scores with the parameters mentioned above to estimate the 10-year risk of developing a cardiovascular event and classify the participants in the groups: less than 5%, 5% to less than 10%, 10% to less than 20%, 20% or greater. With the addition of values of hGH, participants could then be reclassified into different groups. We assessed the reclassification of subjects and calculated a NRI as a measurement of the successfulness of the model. Model discrimination was assessed by calculating the C statistics (Harrell's), which represents the area under the receiver operating characteristics (ROC) curve, for the different models {25}.

All analyses except the NRI and C-statistics were performed using SPSS statistical software (version 20.0.0, SPSS inc, Chicago, Ill). NRI and C-statistics analyses were made with Stata software version 11 (StataCorp, College Station, Texas). A 2-sided P value of less than 0.05 was considered statistically significant.

### Results

### Cross-sectional analysis

Baseline characteristics of the cohort are shown in table 2. Women, as expected, had significantly higher fasting values of hGH than men (table 3). In the crude regression models, all variables were significant determinants of hGH-levels in both genders with the exception of age in women (Table 4). In the adjusted models, age, current smoking and HDL-C had a significant positive correlation with hGH, while LDL-C and BMI exhibited significant negative correlation in both genders (table 4). Systolic blood pressure had a significant negative correlation in females, but not in males. In women diabetes mellitus had a strong negative correlation with hGH in the simple analysis, but was borderline non-significant in the adjusted one. In contrast, the correlation between hGH and diabetes mellitus in males was positive and significant in both models. The multiple regression models had a R² value of 9.5% for males and 11.8% for females. When analyzing the whole cohort and adjusting for gender the R² value was 39%. Addition of the hGH-associated variables waist, insulin (natural logarithm) and body fat percentage rendered a R² value of 10.4% for males and 12.6% for females. Crude regression models including these extra variables all exhibited significant negative correlation with fasting levels of hGH.

### Growth hormone and the risk of CAD

Out of 4358 (1799 males, 2559 females) individuals without a history of previous CAD, 401 (249 males, 152 females) experienced a CAD-event during a median follow-up time of 16.1 years (IQR, 15.4-16.7). Each SD increase of baseline hGH was associated with a multivariate-adjusted HR of 1.14 (P=.008) in the total cohort (Table5). The HR was similar in separate analyses of men (HR, 1.17; p=.01) but not significantly elevated in women. There was no significant sex interaction in the outcome of CAD (p=.37). The top versus the bottom quartile of hGH was associated with a HR of 1.33 (95%CI, 0.99-1.78; p=.05) in the total analysis, 1.46 (95%CI, 1.01-2.09; p=.04) in males and non significant in females (p=.91), (Table 6).

### Growth hormone and risk of stroke (see table 5 and 6)

4417 individuals (1849 males, 2568 females) in the cohort had no previous history of stroke. During a median follow-up time of 16.2 years (IQR, 15.5-16.7), 265 persons (152 males, 123 females) suffered a stroke. Each SD increase of fasting hGH at baseline was associated with a multivariate-adjusted HR of 1.19 in the whole cohort, whereas the gender-specific analyses generated a HR of 1.18 in males and was non-significant in females. There was no significant sex interaction in the outcome (p=.81). The top versus the bottom quartile of hGH was associated with a HR of 1.47 (95%CI, 1.02-2.11; p=.04) in the total analysis, 1.48 (95%CI, 0.91-2.42; p=.11) in males and 1.46 (95% CI, 0.83-2.58; p=.19) in females.

### Growth hormone and total mortality (see table 5 and 6)

Out of 4452 individuals (1872 males, 2580 females) in the cohort, 698 (383 males, 315 females) were deceased after a median follow-up time of 16.2 years (IQR, 15.6-16.7). Each SD increase of fasting hGH at baseline was related to a multivariate-adjusted HR of 1.21 in the whole cohort, corresponding HR for males was 1.26 and non-significant for females. An analysis of sex interaction showed significant difference (p=.01) between the genders in the outcome. The top versus the bottom quartile of hGH was associated with a HR of 1.46 (95%CI, 1.17-1.84; p<.001) in the total analysis, 2.05 (95%CI, 1.47-2.86; p<.001) in males and non significant in females (p=.96).

### Growth Hormone and cardiovascular mortality (table 5 and 6)

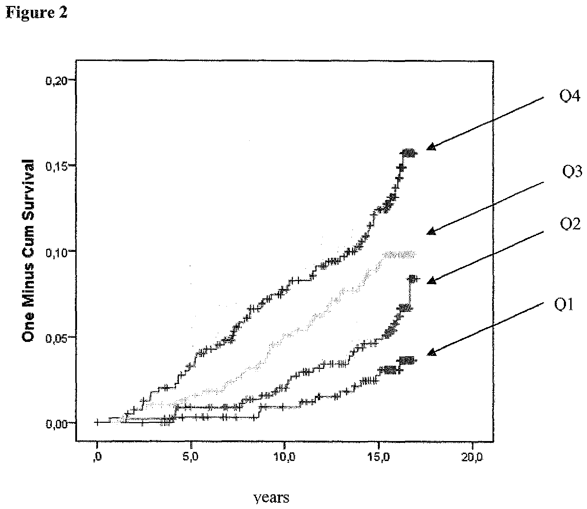

In the cohort 4452 individuals were available for analysis at baseline and during the follow-up time which was identical to the one in total mortality, 215 (126 males, 89 females) of these were deceased with a cardiovascular event as the primary cause of death. Each SD increase of fasting hGH at baseline was associated with a multivariate-adjusted HR of 1.51 in the total cohort. The gender specific HRs were 1.41 for males and 1.38 for females. There was no significant sex interaction in the outcome (p=.81). The top versus the bottom quartile of hGH was associated with a HR of 2.68 (95%CI, 1.70-4.23; p<.001) in the total analysis, 3.41 (95%CI, 1.76-6.61; p<.001) in males and 1.94 (95% CI, 0.98-3.81; p=.06) in females. Figure 2 illustrates the time-dependent development of CVD mortality events in male.

### Growth Hormone is a short term predictor of CVD-mortality.

The time dependent development of CVD mortality is illustrated in Fig.2.

Surprisingly, the predictive discrimination between low risk and high risk individuals was stronger if the observation period was shortened (table 7).
hGH indicates is extraordinary strong in short term risk prediction of CVD mortality in males. The relative risk of events (quartile 4 *vs.* quartile 1 at baseline) is >15 for a 2,5 year risk prediction, 9,1 for a 5 year risk prediction, 7,9 for a 10 year risk prediction and 4,2 for a 15 year risk prediction of CVD mortality in males. These data indicate the strong short term prediction power of hGH and the suitability of serial measurements of hGH.

**Table 7: hGH quartiles for prediction of CVD-mortality in males, variation of observation period. Table 7 illustrates the short term risk of CVD mortality in males. The shorter the observation period, the stronger the relative risk difference between lowest and highest hGH quartiles:**

| | Baseline hGH < 0,06ng/ml (lowest quartile) | Baseline hGH >0,34 ng/ml (highest quartile) | **Relative risk quartile 4/ quartile 1** |
|---|---|---|---|
| 2,5 year follow up | 0 % CVD mortality | 1,2% CVD mortality | **>15** |
| 5 year follow up | 0,35% CVD mortality | 3,2% CVD mortality | **9,1** |
| 10 year follow up | 0,9% CVD mortality | 7,1% CVD mortality | **7,9** |
| 15 year follow up | 2,5% CVD mortality | 10,6% CVD mortality | **4,2** |

### Discrimination and risk reclassification

NRI-analyses were non-significant for CAD and stroke although a trend of improvement could be seen in the total CAD and stroke analysis. In the outcome of total mortality, NRI was significant for males (4.5%; p=0.005), but non-significant in females. Improvement mostly stemmed from down-classification of nonevents (8.6%). In cardiovascular mortality NRI was 11.1% (p<.001) in all subjects and 10.2% (p=.05) in the female cohort, the improvement was especially seen in up-classification of incident events (14.4% of events upgraded in total sample and 16.9% in women).

The addition of hGH to the basic model improved all C-statistic for CAD , stroke, CVD mortality and total mortality. An improvement from 70.5% to 71.4% was seen in the males for total mortality. The largest improvement was seen in CVD-mortality with the c-statistics increasing from 78.7% to 79.8% in the whole cohort, 75.7% to 77.1% in males and 80.4% to 81.2% in females.

**Table 4. Results from multiple linear regression models examining correlations between fasting values of growth hormone and traditional cardiovascular risk markers.**

| | **Male** | | | **Female** | | |
|---|---|---|---|---|---|---|
| Risk marker | B Coefficie nt | 95% CI | P | B Coefficie nt | 95% CI | P |
| Age | 0.02 | 0.01 to 0.03 | <.001 | 0.02 | 0.01 to 0.02 | <.001 |
| Systolic blood pressure Antihypertensive | 0.03 | -0.02 to 0.08 | .19 | -0.04 | -0.08 to 0.00 | .05* |
| medication | 0.12 | 0.00 to 0.24 -0.12 to - | .05 | 0.07 | -0.03 to 0.18 -0.29 to - | .18 |
| BMI | -0.07 | 0.02 | .004 | -0.25 | 0.21 | <.001 |
| Current smoking | 0.30 | 0.20 to 0.40 -0.12 to - | <.001 | 0.19 | 0.10 to 0.27 -0.14 to - | <.001 |
| LDL-C | -0.08 | 0.04 | <.001 | -0.10 | 0.06 | <.001 |
| HDL-C | 0.20 | 0.15 to 0.25 | <.001 | 0.08 | 0.05 to 0.12 | <.001 |
| Diabetes Mellitus | 0.22 | 0.08 to 0.36 | .002 | -0.15 | -0.30 to 0.01 | .06 |

| | | | | | | |
|---|---|---|---|---|---|---|
| The B coefficients are expressed as the increment of standardized values of the natural logarithm of hGH per 1 increment of standardized values (or prescence of dichotomized risk marker) of the risk marker in question. NB age is not standardized. BMI (weight in kilograms divided by height in meters squared), systolic blood pressure, and fasting values of HDL and LDL are standardized. Prevalence of Diabetes mellitus, current smoking and use of antihypertensive medication are dichotomous variables. Abbreviations: BMI, Body Mass Index; LDL-C, Low-density lipoprotein cholesterol; HDL, High-density lipoprotein cholesterol | | | | | | |

**Table 5: Multivariate adjusted Cox proportional hazards model for baseline fasting value of hGH vs. incidence of CAD, stroke, all-cause mortality and cardiovascular mortality.**

| **Event** | Subgroup | n/events | HR | 95% CI | P |
|---|---|---|---|---|---|
| **CAD** | All | 4358/401 | 1.14 | 1.01-1.29 | .04 |
| | Males | 1799/249 | 1.17 | 1.04-1.33 | .01 |
| | Females | 2559/152 | 1.02 | 0.86-1.21 | .81 |
| **Stroke** | All | 4417/265 | 1.19 | 1.02-1.39 | .02 |
| | Males | 1849/152 | 1.18 | 1.01-1.39 | .04 |
| | Females | 2568/123 | 1.14 | 0.94-1.39 | .18 |
| **Total mortality** | All | 4452/698 | 1.21 | 1.11-1.33 | <.001 |
| | Males | 1872/383 | 1.26 | 1.14-1.39 | <.001 |
| | Females | 2580/315 | 1.04 | 0.92-1.17 | .53 |
| **CVD mortality** | All | 4452/215 | 1.51 | 1.28-1.78 | <.001 |
| | Males | 1872/126 | 1.41 | 1.20-1.66 | <.001 |
| | Females | 2580/89 | 1.38 | 1.08-1.76 | .009 |

| | | | | | |
|---|---|---|---|---|---|
| Hazard ratios (HR) (95% CI) are expressed per 1 SD increment of the natural logarithm of hGH. Variables adjusted for in the analysis: age, systolic blood pressure, use of antihypertensive medication, BMI (weight in kilograms divided by height in meters squared), Prevalence of diabetes mellitus, current smoking and fasting values of HDL and LDL. In addition adjusted for sex in the gender combined analyses. | | | | | |

### Quartile Analysis

**Table 6. Cox regression hGH. Quartiles.**

| | | | Q1 | | Q2 | | | Q3 | | | Q4 | | | Trend | | |
|---|---|---|---|---|---|---|---|---|---|---|---|---|---|---|---|---|
| Event | n/events | M/F | HR | P | HR | 95 % CI | P | HR | 95% CI | P | HR | 95 % CI | P | HR | 95 % CI | P |
| CAD | 4358/401 | 1799/2559 | 1 (ref) | .21 | 1,097 | 0.83-1.46 | .52 | 1,057 | 0.78-1.43 | .72 | 1,331 | 0.99-1.78 | .05 | 1,088 | 0.99-1.20 | .08 |
| | 1799/249 | M | 1 (ref) | .02 | ,923 | 0.64-1.33 | .67 | ,938 | 0.63-1.39 | .75 | 1,457 | 1.01-2.09 | .04 | 1,145 | 1.02-1.29 | .03 |
| | 2559/152 | F | 1 (ref) | .45 | 1,374 | 0.88-2.14 | .16 | 1,241 | 0.78-1.98 | .37 | 1,032 | 0.62-1.73 | .91 | 1,002 | 0.86-1.17 | .98 |
| Stroke | 4417/265 | 1849/2568 | 1 (ref) | .22 | 1,181 | 0.82-1.70 | .37 | 1,279 | 0.88-1.85 | .19 | 1,466 | 1.02-2.11 | .04 | 1,129 | 1.01-1.27 | .04 |
| | 1849/142 | M | 1 (ref) | .19 | 1,077 | 0.66-1.77 | .77 | ,938 | 0.55-1.61 | .82 | 1,482 | 0.91-2.42 | .11 | 1,130 | 0.97-1.32 | .13 |
| | 2568/123 | F | 1 (ref) | .21 | 1,294 | 0.76-2.21 | .34 | 1,736 | 1.04-2.91 | .04 | 1,460 | 0.83-2.58 | .19 | 1,154 | 0.97-1.37 | .10 |
| Total mortality | 4452/698 | 1872/2580 | 1 (ref) | <.001 | ,964 | 0.76-1.22 | .76 | 1,342 | 1.07-1.69 | .01 | 1,463 | 1.17-1.84 | <.001 | 1,165 | 1.09-1.25 | <.001 |
| | 1872/383 | M | 1 (ref) | <.001 | 1,220 | 0.86-1.73 | .27 | 1,756 | 1.25-2.48 | .001 | 2,049 | 1.47-2.86 | <.001 | 1,283 | 1.16-1.42 | <.001 |
| | 2580/315 | F | 1 (ref) | .24 | ,785 | 0.57-1.09 | .15 | 1,086 | 0.80-1.48 | .60 | ,992 | 0.71-1.38 | .96 | 1,032 | 0.93-1.15 | .56 |
| CVD mortality | 4452/215 | 1872/2580 | 1 (ref) | <.001 | 1,391 | 0.85-2.27 | .19 | 2,346 | 1.48-3.72 | <.001 | 2,677 | 1.70-4.23 | <.001 | 1,393 | 1.22-1.59 | <.001 |
| | 1872/126 | M | 1 (ref) | <.001 | 1,764 | 0.87-3.57 | .12 | 2,702 | 1.36-5.37 | .005 | 3,410 | 1.76-6.61 | <.001 | 1,454 | 1.22-1.73 | <.001 |
| | 2580/89 | F | 1 (ref) | .03 | 1,022 | 0.51-2.06 | .95 | 2,050 | 1.09-3.86 | .03 | 1,936 | 0.98-3.81 | .06 | 1,310 | 1.06-1.61 | .01 |

| | | | | | | | | | | | | | | | | |
|---|---|---|---|---|---|---|---|---|---|---|---|---|---|---|---|---|
| Q1-Q4 represents the different quartiles, where Q1 is the 25% of the cohort with the lowest hGH-value. HR in Q1-Q4 is expressed *vs*. the HR in Q1 (reference) which is set to 1.00. Quartiles are gender-specific, ie divided into quartiles separately for men and women, which makes male/female ratio the same in all cohorts, but the cutoff-values different in men and women (see also table 3). Trend is a standard cox proportional hazards analysis where the HR is expressed per increment of quartile. The cut off concentrations for the different quartiles is given in table 3. | | | | | | | | | | | | | | | | |

### Figure description

**Figure 1****:** shows a typical us-hGH assay dose/ signal curve.
**Figure 2****:** Kaplan Meier Analysis of hGH quartiles (see table 3) of CVD mortality prediction in males. Y-axis: One minus Survival Functions (% event/ 100)X-axis: Follow-up period (years) form baseline to emigration or death or last follow-up date (2009-06-30).

### Literature

1. Møller N, Jorgensen JOL. Effects of Growth Hormone on Glucose, Lipid, and Protein Metabolism in Human Subjects. Endocrine reviews. 2009;30(2):152-77.
2. Rosen T, Bengtsson BA. Premature mortality due to cardiovascular disease in hypopituitarism. Lancet. 1990;336(8710):285-8. Epub 1990/08/04.
3. Svensson J, Bengtsson BA, Rosen T, Oden A, Johannsson G. Malignant disease and cardiovascular morbidity in hypopituitary adults with or without growth hormone replacement therapy. The Journal of clinical endocrinology and metabolism. 2004;89(7):3306-12. Epub 2004/07/09.
4. Bulow B, Hagmar L, Mikoczy Z, Nordstrom CH, Erfurth EM. Increased cerebrovascular mortality in patients with hypopituitarism. Clinical endocrinology. 1997;46(1):75-81. Epub 1997/01/01.
5. Tomlinson JW, Holden N, Hills RK, Wheatley K, Clayton RN, Bates AS, et al. Association between premature mortality and hypopituitarism. West Midlands Prospective Hypopituitary Study Group. Lancet. 2001;357(9254):425-31. Epub 2001/03/29.
6. Abs R, Feldt-Rasmussen U, Mattsson AF, Monson JP, Bengtsson BA, Goth MI, et al. Determinants of cardiovascular risk in 2589 hypopituitary GH-deficient adults - a KIMS database analysis. European journal of endocrinology / European Federation of Endocrine Societies. 2006;155(1):79-90. Epub 2006/06/24.
7. de Boer H, Blok GJ, Voerman HJ, Phillips M, Schouten JA. Serum lipid levels in growth hormone-deficient men. Metabolism: clinical and experimental. 1994;43(2):199-203. Epub 1994/02/01.
8. Bulow B, Hagmar L, Eskilsson J, Erfurth EM. Hypopituitary females have a high incidence of cardiovascular morbidity and an increased prevalence of cardiovascular risk factors. The Journal of clinical endocrinology and metabolism. 2000;85(2):574-84. Epub 2000/02/26.
9. Sesmilo G, Biller BM, Llevadot J, Hayden D, Hanson G, Rifai N, et al. Effects of growth hormone administration on inflammatory and other cardiovascular risk markers in men with growth hormone deficiency. A randomized, controlled clinical trial. Annals of internal medicine. 2000;133(2):111-22. Epub 2000/07/15.
10. Beauregard C, Utz AL, Schaub AE, Nachtigall L, Biller BM, Miller KK, et al. Growth hormone decreases visceral fat and improves cardiovascular risk markers in women with hypopituitarism: a randomized, placebo-controlled study. The Journal of clinical endocrinology and metabolism. 2008;93(6):2063-71. Epub 2008/04/03.
11. Maison P, Griffin S, Nicoue-Beglah M, Haddad N, Balkau B, Chanson P. Impact of growth hormone (GH) treatment on cardiovascular risk factors in GH-deficient adults: a Metaanalysis of Blinded, Randomized, Placebo-Controlled Trials. The Journal of clinical endocrinology and metabolism. 2004;89(5):2192-9. Epub 2004/05/06.
12. Orme SM, McNally RJ, Cartwright RA, Belchetz PE. Mortality and cancer incidence in acromegaly: a retrospective cohort study. United Kingdom Acromegaly Study Group. The Journal of clinical endocrinology and metabolism. 1998;83(8):2730-4. Epub 1998/08/26.
13. Melmed S, Casanueva FF, Klibanski A, Bronstein MD, Chanson P, Lamberts SW, et al. A consensus on the diagnosis and treatment of acromegaly complications. Pituitary. 2012. Epub 2012/08/21.
14. Colao A, Marzullo P, Di Somma C, Lombardi G. Growth hormone and the heart. Clinical endocrinology. 2001;54(2): 137-54. Epub 2001/02/24.
15. Maison P, Tropeano AI, Macquin-Mavier I, Giustina A, Chanson P. Impact of somatostatin analogs on the heart in acromegaly: a metaanalysis. The Journal of clinical endocrinology and metabolism. 2007;92(5): 1743-7. Epub 2007/02/22.
16. Takala J, Ruokonen E, Webster NR, Nielsen MS, Zandstra DF, Vundelinckx G, et al. Increased mortality associated with growth hormone treatment in critically ill adults. The New England journal of medicine. 1999;341(11):785-92. Epub 1999/09/09.
17. Maison P, Balkau B, Simon D, Chanson P, Rosselin G, Eschwege E. Growth hormone as a risk for premature mortality in healthy subjects: data from the Paris prospective study. BMJ (Clinical research ed). 1998;316(7138): 1132-3. Epub 1998/04/29.
18. Berglund G, Elmstahl S, Janzon L, Larsson SA. The Malmo Diet and Cancer Study. Design and feasibility. Journal of internal medicine. 1993;233(1):45-51. Epub 1993/01/01.
19. Rosvall M, Janzon L, Berglund G, Engstrom G, Hedblad B. Incidence of stroke is related to carotid IMT even in the absence of plaque. Atherosclerosis. 2005;179(2):325-31. Epub 2005/03/22.
20. Bidlingmaier M, Suhr J, Ernst A, Wu Z, Keller A, Strasburger CJ, et al. High-sensitivity chemiluminescence immunoassays for detection of growth hormone doping in sports. Clinical chemistry. 2009;55(3):445-53. Epub 2009/01/27.
21. Jerntorp P, Berglund G. Stroke registry in Malmö, Sweden. Stroke. 1992;23(3):357-61.
22. Hammar N, Alfredsson L, Rosen M, Spetz CL, Kahan T, Ysberg AS. A national record linkage to study acute myocardial infarction incidence and case fatality in Sweden. International Journal of Epidemiology. 2001;30(suppl 1):S30.
23. Ludvigsson J, Andersson E, Ekbom A, Feychting M, Kim J-L, Reuterwall C, et al. External review and validation of the Swedish national inpatient register. BMC Public Health. 2011;11(1):450.
24. D'Agostino RB, Sr., Vasan RS, Pencina MJ, Wolf PA, Cobain M, Massaro JM, et al. General cardiovascular risk profile for use in primary care: the Framingham Heart Study. Circulation. 2008;117(6):743-53. Epub 2008/01/24.
25. Pencina MJ, D'Agostino RB, Sr., D'Agostino RB, Jr., Vasan RS. Evaluating the added predictive ability of a new marker: from area under the ROC curve to reclassification and beyond. Statistics in medicine. 2008;27(2): 157-72; discussion 207-12. Epub 2007/06/15.
26. Pencina MJ, D'Agostino RB. Overall C as a measure of discrimination in survival analysis: model specific population value and confidence interval estimation. Statistics in medicine. 2004;23(13):2109-23.
27. Engstrom BE, Karlsson FA, Wide L. Marked gender differences in ambulatory morning growth hormone values in young adults. Clinical chemistry. 1998;44(6 Pt 1):1289-95. Epub 1998/06/13.
28. Eden Engstrom B, Karlsson FA, Naessen T, Gillberg P, Wide L. Ambulatory morning growth hormone concentrations increase in men and decrease in women with age. Scandinavian journal of clinical and laboratory investigation. 2002;62(1):25-31. Epub 2002/05/11.
29. Pincus SM, Gevers EF, Robinson IC, van den Berg G, Roelfsema F, Hartman ML, et al. Females secrete growth hormone with more process irregularity than males in both humans and rats. The American journal of physiology. 1996;270(1 Pt 1):E107-15. Epub 1996/01/01.
30. Hindmarsh PC, Dennison E, Pincus SM, Cooper C, Fall CH, Matthews DR, et al. A sexually dimorphic pattern of growth hormone secretion in the elderly. The Journal of clinical endocrinology and metabolism. 1999;84(8):2679-85. Epub 1999/08/12.
31. Veldhuis JD, Roelfsema F, Keenan DM, Pincus S. Gender, age, body mass index, and IGF-I individually and jointly determine distinct GH dynamics: analyses in one hundred healthy adults. The Journal of clinical endocrinology and metabolism. 2011;96(1):115-21. Epub 2010/10/12.
32. Saini S, Hindmarsh PC, Matthews DR, Pringle PJ, Jones J, Preece MA, et al. Reproducibility of 24-hour serum growth hormone profiles in man. Clinical endocrinology. 1991;34(6):455-62. Epub 1991/06/01.
33. Surya S, Symons K, Rothman E, Barkan AL. Complex rhythmicity of growth hormone secretion in humans. Pituitary. 2006;9(2):121-5. Epub 2006/07/18.
34. Jaffe CA, Ocampo-Lim B, Guo W, Krueger K, Sugahara I, DeMott-Friberg R, et al. Regulatory mechanisms of growth hormone secretion are sexually dimorphic. The Journal of clinical investigation. 1998;102(1):153-64. Epub 1998/07/03.
35. Faje AT, Barkan AL. Basal, but not pulsatile, growth hormone secretion determines the ambient circulating levels of insulin-like growth factor-I. The Journal of clinical endocrinology and metabolism. 2010;95(5):2486-91. Epub 2010/03/02.
36. Ho KY, Weissberger AJ. Characterization of 24-hour growth hormone secretion in acromegaly: implications for diagnosis and therapy. Clinical endocrinology. 1994;41(1):75-83. Epub 1994/07/01.
37. van der Klaauw AA, Pereira AM, van Thiel SW, Frolich M, Iranmanesh A, Veldhuis JD, et al. Attenuated pulse size, disorderly growth hormone and prolactin secretion with preserved nyctohemeral rhythm distinguish irradiated from surgically treated acromegaly patients. Clinical endocrinology. 2007;66(4):489-98.
38. Roelfsema F, Biermasz NR, Veldhuis JD. Pulsatile, nyctohemeral and entropic characteristics of GH secretion in adult GH-deficient patients: selectively decreased pulsatile release and increased secretory disorderliness with preservation of diurnal timing and gender distinctions. Clinical endocrinology. 2002;56(1):79-87. Epub 2002/02/19.
39. Bartke A. Can growth hormone (GH) accelerate aging? Evidence from GH-transgenic mice. Neuroendocrinology. 2003;78(4):210-6. Epub 2003/10/30.
40. Coschigano KT, Holland AN, Riders ME, List EO, Flyvbjerg A, Kopchick JJ. Deletion, but not antagonism, of the mouse growth hormone receptor results in severely decreased body weights, insulin, and insulin-like growth factor I levels and increased life span. Endocrinology. 2003;144(9):3799-810. Epub 2003/08/23.
41. Brown-Borg HM, Borg KE, Meliska CJ, Bartke A. Dwarf mice and the ageing process. Nature. 1996;384(6604):33. Epub 1996/11/07.
42. Masternak MM, Bartke A. Growth hormone, inflammation and aging. Pathobiology of aging & age related diseases. 2012;2. Epub 2012/09/07.
43. Guevara-Aguirre J, Balasubramanian P, Guevara-Aguirre M, Wei M, Madia F, Cheng CW, et al. Growth hormone receptor deficiency is associated with a major reduction in pro-aging signaling, cancer, and diabetes in humans. Science translational medicine. 2011;3(70):70ra13. Epub 2011/02/18.
44. Menezes Oliveira JL, Marques-Santos C, Barreto-Filho JA, Ximenes Filho R, de Oliveira Britto AV, Oliveira Souza AH, et al. Lack of evidence of premature atherosclerosis in untreated severe isolated growth hormone (GH) deficiency due to a GH-releasing hormone receptor mutation. The Journal of clinical endocrinology and metabolism. 2006;91(6):2093-9. Epub 2006/03/09.
45. Aguiar-Oliveira MH, Oliveira FT, Pereira RM, Oliveira CR, Blackford A, Valenca EH, et al. Longevity in untreated congenital growth hormone deficiency due to a homozygous mutation in the GHRH receptor gene. The Journal of clinical endocrinology and metabolism. 2010;95(2):714-21. Epub 2009/12/08.
46. Besson A, Salemi S, Gallati S, Jenal A, Horn R, Mullis PS, et al. Reduced longevity in untreated patients with isolated growth hormone deficiency. The Journal of clinical endocrinology and metabolism. 2003;88(8):3664-7. Epub 2003/08/14.
47. Rudling M, Norstedt G, Olivecrona H, Reihner E, Gustafsson JA, Angelin B. Importance of growth hormone for the induction of hepatic low density lipoprotein receptors. Proceedings of the National Academy of Sciences of the United States of America. 1992;89(15):6983-7. Epub 1992/08/01.
48. Rudling M, Olivecrona H, Eggertsen G, Angelin B. Regulation of rat hepatic low density lipoprotein receptors. In vivo stimulation by growth hormone is not mediated by insulin-like growth factor I. The Journal of clinical investigation. 1996;97(2):292-9. Epub 1996/01/15.
49. Vahl N, Klausen I, Christiansen JS, Jorgensen JO. Growth hormone (GH) status is an independent determinant of serum levels of cholesterol and triglycerides in healthy adults. Clinical endocrinology. 1999;51(3):309-16. Epub 1999/09/01.
50. Clasey JL, Weltman A, Patrie J, Weltman JY, Pezzoli S, Bouchard C, et al. Abdominal visceral fat and fasting insulin are important predictors of 24-hour GH release independent of age, gender, and other physiological factors. The Journal of clinical endocrinology and metabolism. 2001;86(8):3845-52. Epub 2001/08/15.
51. Miller KK, Biller BM, Lipman JG, Bradwin G, Rifai N, Klibanski A. Truncal adiposity, relative growth hormone deficiency, and cardiovascular risk. The Journal of clinical endocrinology and metabolism. 2005;90(2):768-74. Epub 2004/12/02.
52. Bredella MA, Lin E, Brick DJ, Gerweck AV, Harrington LM, Torriani M, et al. Effects of GH in women with abdominal adiposity: a 6-month randomized, double-blind, placebo-controlled trial. European journal of endocrinology / European Federation of Endocrine Societies. 2012;166(4):601-11. Epub 2012/01/26.
53. Sandberg H, Roman L, Zavodnick J, Kupers N. The effect of smoking on serum somatotropin, immunoreactive insulin and blood glucose levels of young adult males. The Journal of pharmacology and experimental therapeutics. 1973;184(3):787-91. Epub 1973/03/01.
54. Wilkins JN, Carlson HE, Van Vunakis H, Hill MA, Gritz E, Jarvik ME. Nicotine from cigarette smoking increases circulating levels of cortisol, growth hormone, and prolactin in male chronic smokers. Psychopharmacology. 1982;78(4):305-8. Epub 1982/01/01.
55. Veldhuis JD. Aging and hormones of the hypothalamo-pituitary axis: gonadotropic axis in men and somatotropic axes in men and women. Ageing research reviews. 2008;7(3): 189-208. Epub 2008/03/18.
56. Bramnert M, Segerlantz M, Laurila E, Daugaard JR, Manhem P, Groop L. Growth hormone replacement therapy induces insulin resistance by activating the glucose-fatty acid cycle. The Journal of clinical endocrinology and metabolism. 2003;88(4): 1455-63. Epub 2003/04/08.
57. Norrelund H, Nielsen S, Christiansen J, Jorgensen J, Moller N. Modulation of basal glucose metabolism and insulin sensitivity by growth hormone and free fatty acids during short-term fasting. European Journal of Endocrinology. 2004;150(6):779-87.
58. Ho KY, Evans WS, Blizzard RM, Veldhuis JD, Merriam GR, Samojlik E, et al. Effects of sex and age on the 24-hour profile of growth hormone secretion in man: importance of endogenous estradiol concentrations. The Journal of clinical endocrinology and metabolism. 1987;64(1):51-8. Epub 1987/01/01.
59. Lorenz et al.," Functional Antibodies Targeting IsaA of Staphylococcus aureus Augment Host Immune Response and Open New Perspectives for Antibacterial Therapy"; Antimicrob Agents Chemother. 2011 January; 55(1): 165-173.
60. Lane, R.D. "A short-duration polyethylene glycol fusiontechnique for increasing production of monoclonal antibody-secreting hybridomas", J. Immunol. Meth. 81: 223-228; (1985)
61. Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)

## Claims

1. A method for predicting the cardiovascular risk within 2,5 years in a subject comprising:
▪ determining the fasting level of growth hormone (hGH), and/or its isoforms in a bodily fluid obtained from said subject; and
▪ correlating said fasting level of growth hormone (hGH), and/or its isoforms with a cardiovascular risk, wherein an enhanced level is predictive for an enhanced risk,
wherein the cardiovascular risk is defined as an adverse event and wherein the risk of said adverse event is predicted within 2,5 years and wherein the cardiovascular risk is defined as an adverse event selected from the group comprising stroke, CVD (cardiovascular disease)-mortality and Coronary artery disease (CAD) wherein the latter comprises fatal or nonfatal myocardial infarction, death due to ischemic heart disease, percutaneous coronary intervention (PCI) or coronary artery by-pass grafting (CABG).

2. A method according to claim 1 wherein the risk of CVD mortality is predicted wherein CVD mortality is death because of myocardial infarction, heart failure or stroke.

3. A method according to any of claims 1 or 2 wherein said subject is male.

4. A method according to any of claims 1 to 3 wherein said fasting level of growth hormone (hGH), and/or its isoforms in a bodily fluid is determined by an ultrasensitive assay having an analytical assay sensitivity of less than 100 pg/ml, preferred less than 50 pg/ml, preferred less than 30 pg/ml, preferred less than 20 pg/ml, preferred less than 10 pg/ml, preferred less than 5 pg/ml, preferred 2 pg/ml.

5. A method according to any of claims 1 to 4 wherein said fasting level of growth hormone (hGH), and/or its isoforms in a bodily fluid is determined by an ultrasensitive assay having a functional assay sensitivity of less than 400 pg/ml, preferred less than 200 pg/ml, preferred less than 120 pg/ml, preferred less than 80 pg/ml, preferred less than 40 pg/ml, preferred less than 20 pg/ml, preferred 10 pg/ml most preferred less than 8,5 pg/ml.

6. A method according to any of claims 1 to 5 wherein said assay is either specific for one of the secreted hGH isomers, or for more than one or is specific for and determines all secreted isomers of hGH selected from the group comprising isomer 1, isomer 2, isomer 3 and isomer 4 (SEQ ID. NO: 1 to 4) in said bodily fluid.

7. A method according to any of claims 1 to 6 wherein said a method is performed more than once in order to monitor the cardiovascular risk or the total mortality risk in a subject or in order to monitor the course of treatment.

8. A method according to claim 7 wherein said monitoring is performed in order to evaluate the response of said subject to preventive and/or therapeutic measures taken.

9. A method according to any of claims 1 to 8 in order to stratify said subjects into risk groups and/or to reclassify said subjects for the cardiovascular risk.

10. A method according to any of claims 1-9 wherein the bodily fluid is blood or plasma or serum.

11. A method according to any of claims 1 to 10 wherein said subject is male and wherein the threshold is from 340 pg/ml to 60 pg/ml, wherein a fasting level of growth hormone (hGH), and/or its isoforms above a threshold of 340 pg/ml is predictive for a high risk and a fasting level below a threshold of 60 pg/ml is predictive for a low risk.

12. A method according to any of claims 1 to 10 wherein said subject is female and wherein the threshold is from 3150 pg/ml to 400 pg/ml, wherein a fasting level of growth hormone (hGH), and/or its isoforms above a threshold of 3150 pg/ml is predictive for a high risk and a fasting level below a threshold of 400 pg/ml is predictive for a low risk.

13. A method according to any of claims 1-12, wherein said CVD (cardiovascular disease) is selected from myocardial infarction, heart failure and stroke.

14. A method according to claims 1 to 13, wherein additionally at least one clinical parameter is determined selected from the group comprising: age, presence of diabetes mellitus, current smoking.

15. A method according to any of claims 1 to 14, wherein additionally at least one further biomarker is determined in the bodily fluid of said subject and correlated with said cardiovascular risk, wherein said additional biomarker is selected from the group comprising: pro-Neurotensin (PNT) 1-117 and fragments thereof having at least a length of five amino acids, C-reactive protein (CRP), pro-brain natriuretic peptide 1-108 (pro-BNP) 1-108, Pro-BNP, BNP, Pro Atrial Natriuretic Peptide 1-98 (proANP-N-terminal fragment), Pro-ANP, Adrenomedullin, pro-Adrenomedullin (proADM) 24-71, ProADM 127-164, pro- Atrial Natriuretic Peptide (pro-ANP) and fragments thereof having at least a length of five amino acids, ST-2, GDF15, Galectin-3 and Copeptin.

16. A method according to any of claims 1 to 2, 5 to 10 and 12 to 15, wherein said subject is female and wherein additionally one further biomarker is determined in the bodily fluid of said female subject and correlated with said cardiovascular risk or said total mortality risk, wherein said additional biomarker is pro-Neurotensin (PNT) and fragments thereof having at least a length of five amino acids.

17. A method according to any of claims 1 to 16, wherein said subject has a disease or has had an event selected from the group comprising atherosclerosis, high blood pressure, heart failure, myocardial infarction.

18. A method according to any of claims 1 to 17, wherein the fasting level said of growth hormone (hGH), and/or its isoforms is measured with an immunoassay.

19. A method according to claim 18, wherein said immunoassay comprise at least one antibody, preferably two antibodies, having a binding affinity of 10⁻⁸M or less.

20. A method according to any of claims 18 or 19, wherein said immunoassay is an ultra sensitive hGH assay comprising at least one monospecific binder, preferably two monospecific binders, having a binding affinity of 10⁻⁸M or less to hGH and its isoforms and wherein said binder (s) is either specific for one of the secreted hGH isomers, or for more than one or is specific for and binds to all secreted isomers of hGH selected from the group comprising isomer 1, isomer 2, isomer 3 and isomer 4 (SEQ ID. NO: 1 to 4) and wherein ultrasensitive assay has an analytical assay sensitivity of less than 10 pg/ml, preferred less than 5 pg/ml, preferred 2 pg/ml. or a functional assay sensitivity of less than 20 pg/ml, preferred 10 pg/ml most preferred less than 8,5 pg/ml.

21. A method according to claim 20, wherein said monospecific binder is a monoclonal antibody.

## Patentansprüche

1. Verfahren zur Vorhersage des kardiovaskulären Risikos innerhalb von 2,5 Jahren bei einem Subjekt, umfassend:
▪ Bestimmen des Nüchternwerts des Wachstumshormons (HhH) und/oder dessen Isoformen in einer von diesem Subjekt entnommenen Körperflüssigkeit und
▪ Korrelieren des Nüchternwerts des Wachstumshormons (hGH) und/oder dessen Isoformen mit einem kardiovaskulären Risiko, wobei ein erhöhter Wert prädiktiv für ein erhöhtes Risiko ist,
wobei das kardiovaskuläre Risiko definiert ist als ein unerwünschtes Ereignis und wobei das Risiko des unerwünschten Ereignisses innerhalb von 2,5 Jahren vorhergesagt wird und wobei das kardiovaskuläre Risiko definiert ist als ein unerwünschtes Ereignis ausgewählt aus der Gruppe bestehend aus Schlaganfall, CVD (kardiovaskuläre Erkrankung)-Sterblichkeit und koronarer Herzkrankheit (KHK), wobei Letztere tödlichen oder nicht tödlichen Myokardinfarkt umfasst, Tod durch ischämische Herzkrankheit, perkutaner koronarer Intervention (PCI) oder Koronararterien-Bypass (CABG).

2. Verfahren nach Anspruch 1, wobei das Risiko der CVD-Sterblichkeit vorhergesagt wird, wobei die CVD-Sterblichkeit Tod durch Myokardinfarkt, Herzversagen oder Schlaganfall ist.

3. Verfahren nach einem der Ansprüche 1 oder 2, wobei das Subjekt männlich ist.

4. Verfahren nach einem der Ansprüche 1 bis 3, wobei der Nüchternwert des Wachstumshormons (hGH) und/oder dessen Isoformen in einer Körperflüssigkeit durch einen ultrasensitiven Assay mit einer analytischen Assay-Sensitivität von weniger als 100 pg/ml, bevorzugt weniger als 50 pg/ml, bevorzugt weniger als 30 pg/ml, bevorzugt weniger als 20 pg/ml, bevorzugt weniger als 10 pg/ml, bevorzugt weniger als 5 pg/ml, bevorzugt weniger als 2 pg/ml bestimmt wird.

5. Verfahren nach einem der Ansprüche 1 bis 4, wobei der Nüchternwert des Wachstumshormons (hGH) und/oder dessen Isoformen in einer Körperflüssigkeit durch einen ultrasensitiven Assay mit einer funktionalen Assay-Sensitivität von weniger als 400 pg/ml, bevorzugt weniger als 200 pg/ml, bevorzugt weniger als 120 pg/ml, bevorzugt weniger als 80 pg/ml, bevorzugt weniger als 40 pg/ml, bevorzugt weniger als 20 pg/ml, bevorzugt weniger als 10 pg/ml, am meisten bevorzugt weniger als 8,5 pg/ml bestimmt wird.

6. Verfahren nach einem der Ansprüche 1 bis 5, wobei der Assay entweder für eines der sezernierten hGH-Isomere spezifisch ist, oder für mehr als eines spezifisch ist oder für alle sezernierten Isomere von hGH ausgewählt aus der Gruppe bestehend aus Isomer 1, Isomer 2, Isomer 3 und Isomer 4 (SEQ ID NO: 1 bis 4) in der Körperflüssigkeit spezifisch ist und diese bestimmt.

7. Verfahren nach einem der Ansprüche 1 bis 6, wobei dieses Verfahren mehr als einmal durchgeführt wird, um das kardiovaskuläre Risiko oder das Gesamtsterblichkeitsrisiko bei einem Subjekt zu überwachen oder um den Behandlungsverlauf zu überwachen.

8. Verfahren nach Anspruch 7, wobei die Überwachung durchgeführt wird, um die Reaktion des Subjekts auf getroffene vorbeugende und/oder therapeutische Maßnahmen zu bewerten.

9. Verfahren nach einem der Ansprüche 1 bis 8, um die Subjekte in Risikogruppen zu stratifizieren und/oder um die Subjekte für das kardiovaskuläre Risiko neu einzustufen.

10. Verfahren nach einem der Ansprüche 1 bis 9, wobei die Körperflüssigkeit Blut oder Plasma oder Serum ist.

11. Verfahren nach einem der Ansprüche 1 bis 10, wobei das Subjekt männlich ist und wobei der Schwellenwert zwischen 340 pg/ml und 60 pg/ml liegt, wobei ein Nüchternwert des Wachstumshormons (hGH) und/oder dessen Isoformen über einem Schwellenwert von 340 pg/ml prädiktiv für ein hohes Risiko ist und ein Nüchternwert unter einem Schwellenwert von 60 pg/ml prädiktiv für ein geringes Risiko ist.

12. Verfahren nach einem der Ansprüche 1 bis 10, wobei das Subjekt weiblich ist und wobei der Schwellenwert zwischen 3150 pg/ml und 400 pg/ml liegt, wobei ein Nüchternwert des Wachstumshormons (hGH) und/oder dessen Isoformen über einem Schwellenwert von 3150 pg/ml prädiktiv für ein hohes Risiko ist und ein Nüchternwert unter einem Schwellenwert von 400 pg/ml prädiktiv für ein geringes Risiko ist.

13. Verfahren nach einem der Ansprüche 1 bis 12, wobei die CVD (kardiovaskuläre Erkrankung) ausgewählt ist aus Myokardinfarkt, Herzversagen und Schlaganfall.

14. Verfahren nach den Ansprüchen 1 bis 13, wobei zusätzlich mindestens ein klinischer Parameter bestimmt wird, welcher ausgewählt ist aus der Gruppe bestehend aus Alter, Vorliegen eines Diabetes mellitus, gegenwärtiges Rauchen.

15. Verfahren nach einem der Ansprüche 1 bis 14, wobei zusätzlich mindestens ein weiterer Biomarker in der Körperflüssigkeit des Subjekts bestimmt und mit dem kardiovaskulären Risiko korreliert wird, wobei der zusätzliche Biomarker ausgewählt ist aus der Gruppe bestehend aus: Pro-Neurotensin (PNT) 1-117 und Fragmenten davon mit einer Länge von mindestens fünf Aminosäuren, C-reaktivem Protein (CRP), Pro-Brain natriuretic Peptide 1-108 (proBNP) 1-108, proBNP, BNP, Pro-Atriales natriuretisches Peptid 1-98 (N-terminales Fragment proANP), proANP, Adrenomedullin, Pro-Adrenomedullin (proADM) 24-71, proADM 127-164, Pro-Atriales natriuretisches Peptid (proANP) und Fragmenten davon mit einer Länge von mindestens fünf Aminosäuren, ST-2, GDF15, Galectin-3 und Copeptin.

16. Verfahren nach einem der Ansprüche 1 bis 2, 5 bis 10 und 12 bis 15, wobei das Subjekt weiblich ist und wobei zusätzlich ein weiterer Biomarker in der Körperflüssigkeit des weiblichen Subjekts bestimmt und mit dem kardiovaskulären Risiko oder dem Gesamtsterblichkeitsrisiko korreliert wird, wobei der zusätzliche Biomarker Pro-Neurotensin (PNT) und Fragmente davon mit einer Länge von mindestens fünf Aminosäuren ist.

17. Verfahren nach einem der Ansprüche 1 bis 16, wobei das Subjekt eine Krankheit hat oder ein Ereignis erlitten hat, ausgewählt aus der Gruppe bestehend aus Arteriosklerose, Bluthochdruck, Herzversagen, Myokardinfarkt.

18. Verfahren nach einem der Ansprüche 1 bis 17, wobei der Nüchternwert des Wachstumshormons (hGH) und/oder dessen Isoformen mit einem Immunassay gemessen wird.

19. Verfahren nach Anspruch 18, wobei der Immunassay mindestens einen Antikörper, bevorzugt zwei Antikörper mit einer Bindungsaffinität von 10⁻⁸ M oder weniger umfasst.

20. Verfahren nach einem der Ansprüche 18 oder 19, wobei der Immunassay ein ultrasensitiver hGH-Assay ist, welcher mindestens einen monospezifischen Binder, bevorzugt zwei monospezifische Binder mit einer Bindungsaffinität an hGH und dessen Isoformen von 10⁻⁸ M oder weniger umfasst und wobei der/die Binder entweder für eines der sezernierten hGH-Isomere oder für mehr als eines spezifisch ist/sind oder für alle sezernierten Isomere von hGH, ausgewählt aus der Gruppe bestehend aus Isomer 1, Isomer 2, Isomer 3 und Isomer 4 (SEQ ID NO: 1 bis 4), spezifisch ist/sind und diese bestimmt/bestimmen und wobei der ultrasensitive Assay eine analytische Assay-Sensitivität von weniger als 10 pg/ml, bevorzugt weniger als 5 pg/ml, bevorzugt 2 pg/ml oder eine funktionale Assay-Sensitivität von weniger als 20 pg/ml, bevorzugt 10 pg/ml, am meisten bevorzugt weniger als 8,5 pg/ml hat.

21. Verfahren nach Anspruch 20, wobei der monospezifische Binder ein monoklonaler Antikörper ist.

## Revendications

1. Méthode permettant de prédire le risque cardiovasculaire dans un délai de 2,5 ans chez un sujet, ladite méthode comprenant :
▪ la détermination du taux à jeun d'hormone de croissance (hGH) et/ou de ses isoformes dans un fluide corporel obtenu dudit sujet; et
▪ la mise en corrélation dudit taux à jeun d'hormone de croissance (hGH) et/ou de ses isoformes avec un risque cardiovasculaire, un taux accru étant prédictif d'un risque accru,
le risque cardiovasculaire étant défini comme un événement indésirable et le risque dudit événement indésirable étant prédit dans un délai de 2,5 ans et le risque cardiovasculaire étant défini comme un événement indésirable choisi dans le groupe comprenant l'accident vasculaire cérébral, la mortalité due aux maladies cardiovasculaires (MCV) et la maladie coronarienne (MC), cette dernière comprenant l'infarctus du myocarde mortel ou non mortel, le décès dû à la cardiopathie ischémique, l'intervention coronarienne percutanée (ICP) ou la greffe de pontage d'artère coronaire (GPAC).

2. Méthode selon la revendication 1, le risque de mortalité due aux MCV étant prédit, dans laquelle la mortalité par MCV est la mort due à l'infarctus du myocarde, à l'insuffisance cardiaque ou à l'accident vasculaire cérébral.

3. Méthode selon l'une quelconque des revendications 1 à 2, ledit sujet étant masculin.

4. Méthode selon l'une quelconque des revendications 1 à 3, ledit taux à jeun d'hormone de croissance (hGH) et/ou de ses isoformes dans un fluide corporel étant déterminé par un test ultrasensible ayant une sensibilité analytique inférieure à 100 pg/ml, de préférence inférieure à 50 pg/ml, de préférence inférieure à 30 pg/ml, de préférence inférieure à 20 pg/ml, de préférence inférieure à 10 pg/ml, de préférence inférieure à 5 pg/ml, de préférence de 2 pg/ml.

5. Méthode selon l'une quelconque des revendications 1 à 4, ledit taux à jeun d'hormone de croissance (hGH) et/ou de ses isoformes dans un fluide corporel étant déterminé par un test ultrasensible ayant une sensibilité de test fonctionnel inférieure à 400 pg/ml, de préférence inférieure à 200 pg/ml, de préférence inférieure à 120 pg/ml, de préférence inférieure à 80 pg/ml, de préférence inférieure à 40 pg/ml, de préférence inférieure à 20 pg/ml, de préférence de 10 pg/ml, de manière la plus préférée inférieure à 8,5 pg/ml.

6. Méthode selon l'une quelconque des revendications 1 à 5, ledit test étant spécifique pour l'un des isomères sécrétés de la hGH ou pour plus d'un de ceux-ci ou alors étant spécifique pour et déterminant tous les isomères sécrétés de la hGH choisis dans le groupe comprenant l'isomère 1, l'isomère 2, l'isomère 3 et l'isomère 4 (SEQ ID n° 1 à 4) dans ledit fluide corporel.

7. Méthode selon l'une des revendications 1 à 6, ladite méthode étant réalisée plus d'une fois pour surveiller le risque cardiovasculaire ou le risque total de mortalité chez un sujet ou pour surveiller le déroulement du traitement.

8. Méthode selon la revendication 7, ladite surveillance étant effectuée pour évaluer la réponse dudit sujet aux mesures préventives et/ou thérapeutiques prises.

9. Méthode selon l'une des revendications 1 à 8 permettant de classer lesdits sujets en groupes à risque et/ou de reclasser lesdits sujets en fonction du risque cardiovasculaire.

10. Méthode selon l'une des revendications 1 à 9, le fluide corporel étant le sang, le plasma ou le sérum.

11. Méthode selon l'une quelconque des revendications 1 à 10, ledit sujet étant masculin et le seuil variant entre 340 pg/ml et 60 pg/ml, un taux à jeun d'hormone de croissance (hGH) et/ou de ses isoformes supérieur à un seuil de 340 pg/ml étant prédictif d'un risque élevé et un taux à jeun inférieur à un seuil de 60 pg/ml étant prédictif d'un risque faible.

12. Méthode selon l'une quelconque des revendications 1 à 10, ledit sujet étant féminin et le seuil variant entre 3150 pg/ml et 400 pg/ml, un taux à jeun d'hormone de croissance (hGH) et/ou de ses isoformes supérieur à un seuil de 3150 pg/ml étant prédictif d'un risque élevé et un taux à jeun inférieur à un seuil de 400 pg/ml étant prédictif d'un risque faible.

13. Méthode selon l'une des revendications 1 à 12, ladite maladie cardiovasculaire (MCV) étant choisie parmi l'infarctus du myocarde, l'insuffisance cardiaque et l'accident vasculaire cérébral.

14. Méthode selon les revendications 1 à 13, au moins un paramètre clinique étant en outre déterminé et sélectionné dans le groupe comprenant : l'âge, la présence d'un diabète sucré, le tabagisme actuel.

15. Méthode selon l'une quelconque des revendications 1 à 14, au moins un autre biomarqueur étant en outre déterminé dans le fluide corporel dudit sujet et mis en corrélation avec ledit risque cardiovasculaire, ledit biomarqueur additionnel étant choisi dans le groupe comprenant : pro-neurotensine (PNT) 1-117 et ses fragments ayant au moins une longueur de cinq acides aminés, protéine C réactive (CRP), peptide natriurétique pro-cérébral 1-108 (pro-BNP) 1-108, pro-BNP, BNP, peptide natriurétique pro-atrial 1-98 (fragment N-terminal du proANP), pro-ANP, adrénomédulline, pro-adrénomédulline (proADM) 24-71, proADM 127-164, peptide natriurétique pro-atrial (pro-ANP) et leurs fragments ayant au moins une longueur de cinq acides aminés, ST-2, GDF15, galectine-3 et copeptine.

16. Méthode selon l'une quelconque des revendications 1 à 2, 5 à 10 et 12 à 15, ledit sujet étant féminin et un autre biomarqueur étant en outre déterminé dans le fluide corporel dudit sujet féminin et mis en corrélation avec ledit risque cardiovasculaire ou ledit risque total de mortalité, ledit biomarqueur supplémentaire étant la pro-neurotensine (PNT) et ses fragments ayant au moins une longueur de cinq acides aminés.

17. Méthode selon l'une quelconque des revendications 1 à 16, ledit sujet étant atteint d'une maladie ou ayant eu un événement choisi dans le groupe comprenant l'athérosclérose, l'hypertension, l'insuffisance cardiaque, l'infarctus du myocarde.

18. Méthode selon l'une des revendications 1 à 17, le taux à jeun de ladite hormone de croissance (hGH) et/ou de ses isoformes étant mesuré à l'aide d'un test immunologique.

19. Méthode selon la revendication 18, ledit test immunologique comprenant au moins un anticorps, de préférence deux anticorps, ayant une affinité de liaison de 10⁻⁸M ou moins.

20. Méthode selon l'une quelconque des revendications 18 ou 19, ledit test immunologique étant un test de hGH ultrasensible comprenant au moins un liant monospécifique, de préférence deux liants monospécifiques, ayant une affinité de liaison de 10⁻⁸M ou moins pour la hGH et ses isoformes et ledit ou lesdits liants étant spécifiques pour l'un des isomères sécrétés de la hGH ou pour plus d'un de ceux-ci, ou alors étant spécifiques pour et se liant à tous les isomères sécrétés de la hGH choisis dans le groupe comprenant l'isomère 1, l'isomère 2, l'isomère 3 et l'isomère 4 (SEQ ID n° 1 à 4) et le test ultrasensible ayant une sensibilité de test analytique inférieure à 10 pg/ml, de préférence inférieure à 5 pg/ml, de préférence de 2 pg/ml ou une sensibilité de test fonctionnel inférieure à 20 pg/ml, de préférence de 10 pg/ml, de manière la plus préférée inférieure à 8,5 pg/ml.

21. Méthode selon la revendication 20, ledit liant monospécifique étant un anticorps monoclonal.
